# EUROPEAN PATENT APPLICATION

(11) **EP 3 338 742 A1**
(43) Date of publication of application: **27.06.2018**
(21) Application number: 18154476.8
(22) Date of filing: 25.01.2010
(51) Int. Cl.: A61F 5/01, A44B 11/25

(54) **ADJUSTABLE MODULAR KNEE BRACE**

(30) Priority: 03.02.2009 AU 2009900392
(62) Divisional of application: 10738141.0
(71) Applicant: Pod I.P. Pty Ltd, Geelong, Victoria 3220 (AU)
(72) Inventor: MALONEY, Geoff, Geelong, Victoria 3220 (AU)
(74) Representative: Boult Wade Tennant

(57) **Abstract**

A system comprises a knee brace (10) including a strap (56) and an apparatus for fastening the strap to the knee brace. The apparatus comprises a tab (24) including a keyhole slot (64) and an elongate slot (62) and an embedded pin (60) protruding from the knee brace and terminating in an enlarged head. The keyhole slot comprises first, second and third slot portions, the first being larger than the second and third, and the third being larger than the second. As the pin traverses from the first slot portion to the third slot portion via the narrow second slot portion, the tab is rotatably secured to the knee brace. The narrowest second slot portion inhibits movement of the pin from the third slot portion back to the first slot portion. The strap is threadable through the elongate slot and rotatably movable relative to the knee brace via the tab.

## Description

### FIELD OF THE INVENTION

The present invention relates to an adjustable modular knee brace.

### BACKGROUND OF THE INVENTION

Previously proposed knee braces generally have complicated constructions which prevent their fit and function from being easily adjusted and customised for different users and/or uses.

It is desirable to provide a knee brace having a simple modular construction that allows the fit and function of the knee brace to be easily adjusted and customised for different users and/or uses.

### SUMMARY OF THE INVENTION

According to the present invention, there is provided a knee brace including upper and lower pairs of struts articulately interconnected by two ligaments respectively located inside two joint capsules, wherein the upper and lower pairs of struts are respectively removably connected to opposite ends of the two ligaments by upper and lower removable fasteners located inside the two joint capsules, and wherein each joint capsule has a hole to access one of the upper and lower fasteners thereby allowing each of the upper and lower pairs of struts to be disconnected from one another by a single fastener.

Upper and lower cuffs can be respectively integrally formed with the upper and lower pairs of struts.

Alternatively, the upper and lower cuffs can be respectively removably connected to co-joined pairs of upper and lower struts.

The upper and lower cuffs can be respectively removably connected to the co-joined pairs of upper and lower struts by notch-pins mounted in the co-joined pairs of upper and lower struts to respectively engage keyholes in the upper and lower cuffs.

Upper and lower cuff adjustment tabs can be respectively removably connected to the upper and lower cuffs to respectively conform the upper and lower cuffs to a leg above and below a knee.

The upper and lower cuff adjustment tabs can be respectively removably connected to the upper and lower cuffs by notch-pins mounted in the upper and lower cuffs to respectively engage keyholes in the upper and lower cuff adjustment tabs.

Strap tabs can be respectively removably connected to the upper and lower pairs of struts to receive straps.

The strap tabs can be respectively removably connected to the upper and lower pairs of struts by notch-pins mounted in the upper and lower pairs of struts to respectively engage keyholes in the strap tabs.

### BRIEF DESCRIPTION OF THE DRAWINGS

The invention will be further described by way of example only with reference to the accompanying drawings, in which:
Figure 1 is a perspective view of an embodiment of adjustable modular knee brace of the invention;
Figure 2 is an exploded perspective view of an articulated joint of the knee brace;
Figures 3 to 5 are partial sectional views of the articulated joint in use;
Figures 6 to 9 are exploded perspective views of a ligament of the articulated joint;
Figure 10 is a perspective view of a snap-fit patella guard of the knee brace;
Figures 11 to 13 are cross sections of sequential snap fitting of the patella guard to the knee brace;
Figures 14(a) to (c) are perspective detail views showing sequential connection of a strap tab of the knee brace;
Figures 15 to 17 are cross sections of different cuff adjustment tabs mounted to the knee brace;
Figures 18 and 19 are front and side views of the knee brace; and
Figures 20 to 22 are front views of different modular cuffs and co-joined struts of the knee brace.

### DETAILED DESCRIPTION

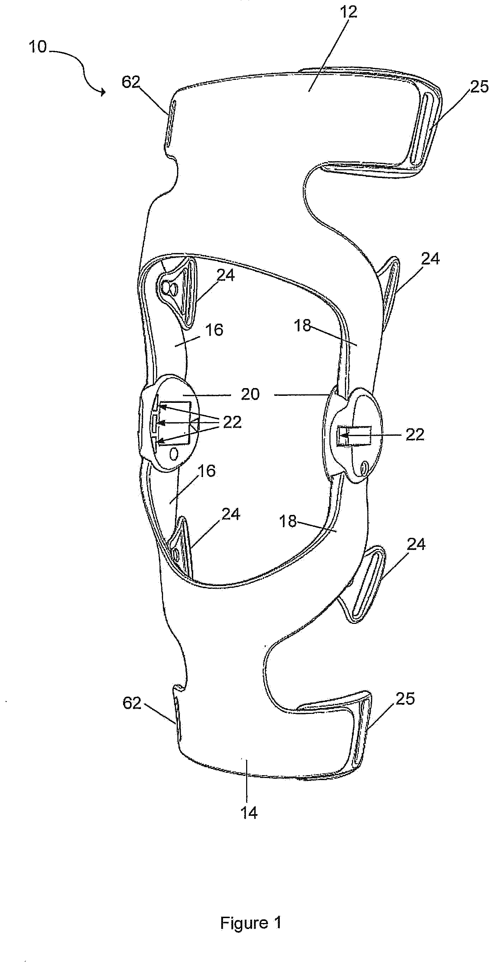

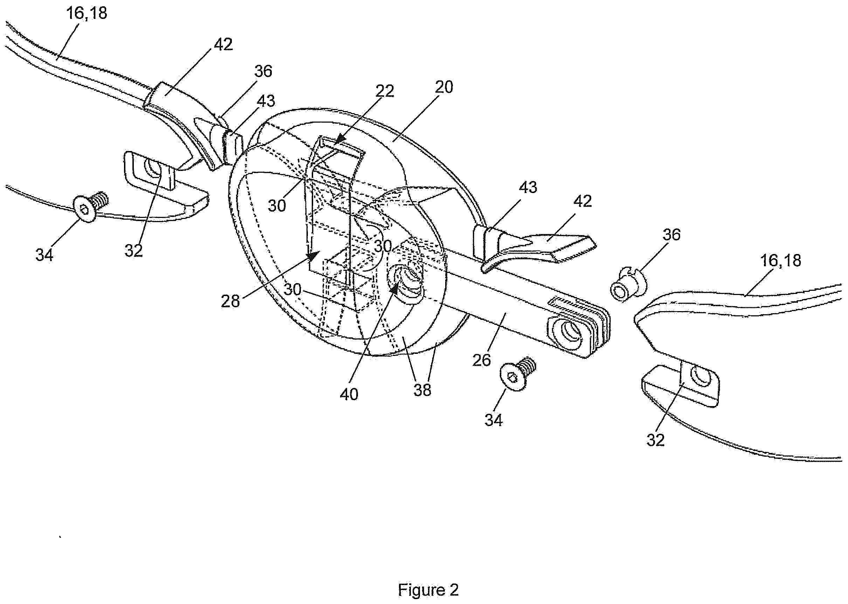

Figures 1 and 2 illustrate one embodiment of a knee brace 10, for example a rehabilitation knee brace or a functional knee brace, of the invention. The knee brace 10 includes upper and lower pairs of struts 16, 18 articulately interconnected by two ligaments 26 respectively located inside two joint capsules 20. The upper and lower pairs of struts 16, 18 are respectively removably connected to opposite ends of the two ligaments 26 by upper and lower removable fasteners 34, 36, for example screws and threaded sleeves, located inside the two joint capsules 20. Each capsule 20 has a hole 40 to access one of the upper and lower fasteners 34, 36 thereby allowing each of the upper and lower pairs of struts 16, 18 to be disconnected from one another by a single fastener. The knee brace 10 further includes upper and lower cuffs 12, 14 are respectively integrally formed, for example as integral mouldings in composite materials, with the upper and lower pairs of struts 16, 18. The joint capsules 20 are formed, for example, as integral mouldings in plastics.

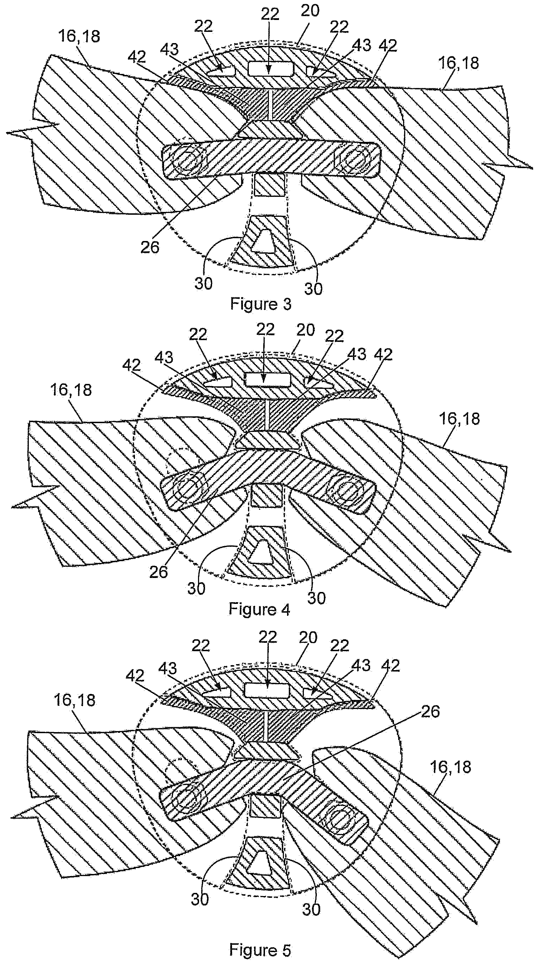

Referring to Figures 2 to 5, each ligament 26 passes through a channel 28 between opposite facing articulation surfaces 30 inside a joint capsule 20. The opposed ends of the upper and lower pairs of struts 16, 18 respectively have a recessed mounting plate 32, and opposite ends of the ligament 26 are respectively removably mounted to the recessed mounting plates 32 by the screws 34 and threaded sleeves 36. The joint capsule 20 has a pair of spaced side flanges 38 which surround the opposed ends of the upper and lower pairs of struts 16, 18, the ligament 26, and the articulation surfaces 30. This ensures that the component parts of the articulated joints of the knee brace 10 are protected by the joint capsules 20 from being dislodged and lost during use. The side flanges 38 have, for example, a generally planar ovoid shape. A single through-hole 40 is provided in the side flanges 38 of the joint capsule 20 to allow external access to one of the screws 34 removably mounting the ligament 26. This allows each of the upper and lower pairs of struts 16, 18 to be disconnected from one another by a single fastener, which in turn allows simple replacement and/or interchange of the ligament 26.

In use, the ligament 26 holds the opposed ends of the upper and lower pairs of struts 16, 18 in mutual articulatory bearing engagement with the corresponding opposite facing articulation surfaces 30 inside the joint capsule 20. The ligament 26 resiliently restrains and dampens flexion and extension of the knee brace 10. The opposite facing articulation surfaces 30 inside the joint capsule 20 provide hyperflexion and hyperextension stops. Removable resilient articulation inserts 42 are superimposably and removably mounted on the opposite facing articulation surfaces 30, via single posts 43 on the inserts 42 and holes in the surfaces 30, to selectively resiliently limit and dampen flexion and extension, and to selectively vary the hyperflexion and hyperextension stops. The resilient inserts 42 are formed, for example, as mouldings in resilient, shock-absorbing (or shock-attenuating) plastics. The overlapping side flanges 38 of the joint capsule 20 regulate medial-lateral and rotational movement of the upper and lower pairs of struts 16, 18 and cuffs 12, 14 during flexion and extension. The articulated joints of the knee brace 10 formed by the above components are substantially silent in operation.

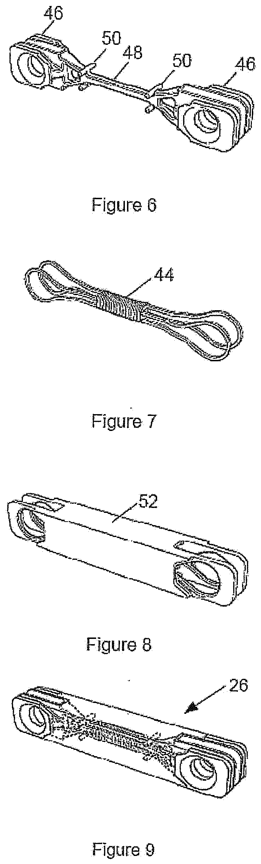

Referring to Figures 6 to 9, each ligament 26 is formed by a tensioned filament 44 looped over a spaced pair of spools 46, and wound radially on an elongate inter-spool segment 48. Catches 50 are formed at the junctions of the spools 46 and inter-spool segment 48 to radially constrain the filament 44 to the inter-spool segment 48. The ligament 26 further includes a resilient overmoulding 52 over the inter-spool segment 48, the radially wound portions of the filament 44, and side edges of the spools 46. The filament 44 is, for example, a manufactured fibre. The spools 46 and inter-spool segment 48 are formed, for example, as an integral moulding in plastics, and the overmoulding 52 is, for example, an overmoulding in resilient, shock-absorbing (or shock-attenuating) plastics. The characteristics of the ligament 26 are influenced by the quantity of fibre 44 (i.e. the number of loops) and the hardness of the overmoulding 52.

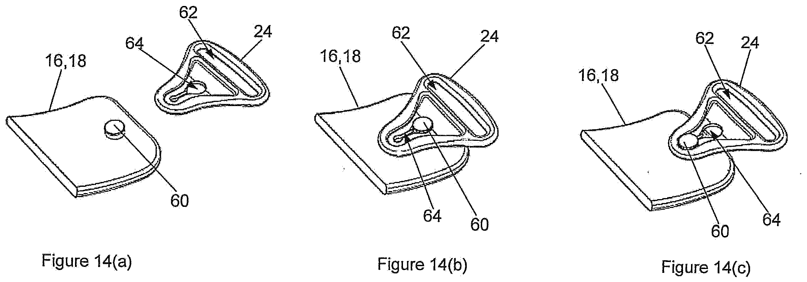

Referring to Figures 1, 14(a) to (c), 18, and 19, strap tabs 24 are respectively removably connected to the upper and lower pairs of struts 16, 18. The strap tabs 25 respectively have elongate slots 62 to receive straps 56. The straps 56 are adjustable, for example, via hook and loop fasteners. Figures 14(a) to (c) sequentially illustrate connection of the strap tabs 24. to the upper and lower pairs of struts 16, 18. The strap tabs 24 are, for example, respectively removably connected to the upper and lower pairs of struts 16, 18 by notch-pins 60 mounted, for example mouldingly embedded, in the upper and lower pairs of struts 16, 18 to respectively engage keyholes 64 in the strap tabs 24. The benefits of the notch-pin 60 and keyhole 64 system are tool-less connectivity and ease of use over the alternatives of fasteners such as rivets, screws, and nuts.

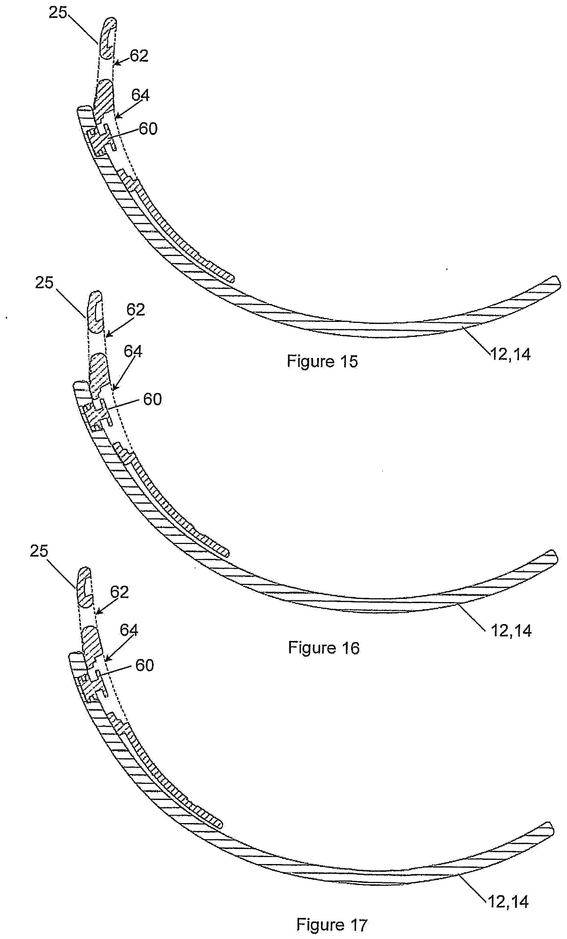

Referring to Figures 1 and 15 to 19, upper and lower cuff adjustment tabs 25 are respectively removably connected one side, for example the lateral side, of the upper and lower cuffs 12, 14 to respectively conform the upper and lower cuffs 12, 14 to a.leg (not shown) above and below a knee. The upper and lower cuff adjustment tabs 25 respectively have elongate slots 62 to receive straps 56. The straps 56 are also received in elongate slots 62 in the other side, for example the medial side, of the upper and lower cuffs 12, 14. Referring to Figures 15 to 17, the upper and lower cuff adjustment tabs 25 are, for example, respectively removably connected to the lateral side of the upper and lower cuffs 12, 14 in similar fashion to the strap tabs 24, namely by notch-pins 60 mounted, for example mouldingly embedded, in the upper and lower cuffs 12, 14 to respectively engage keyholes 64 in the upper and lower cuff adjustment tabs 25. Figures 15 to 17 illustrate different shaped and curved cuff adjustment tabs 25 to adjust the fit of the upper and lower cuffs 12, 14 to the contours of the leg (not shown). For example, the cuff adjustment tabs 25 illustrated in Figure 15 to 17 have decreasing amounts of inward curvature to adjust the fit of the upper and lower cuffs 12, 14 differently contoured legs (not shown). The strap tabs 24 and the cuff adjustment tabs 25 are formed, for example, as mouldings in plastics, for example, rigid nylon. The notch-pins 60 are, for example, made of metal.

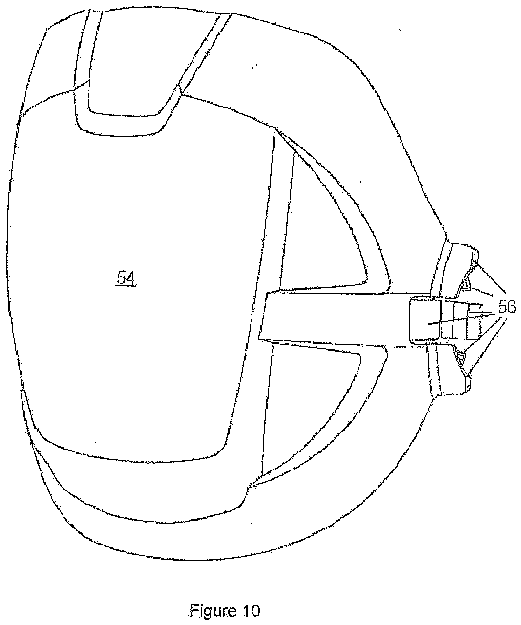

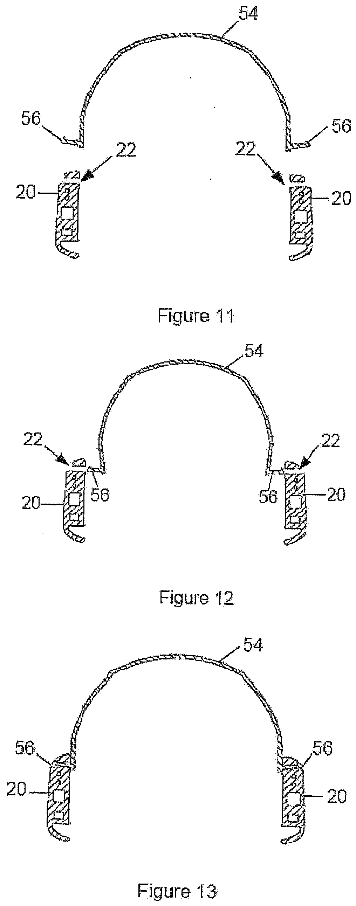

Referring to Figure 1, 10 to 13, 18, and 19, the knee brace 10 optionally further includes a snap-fit patella guard 54 having medial and lateral posterior tongues and side projections 56 that respectively snap fit in and around the anterior transverse holes 22 provided in the medial and lateral joint housings 20. The patella guard 54 is formed, for example, as an integral moulding in resilient plastics.

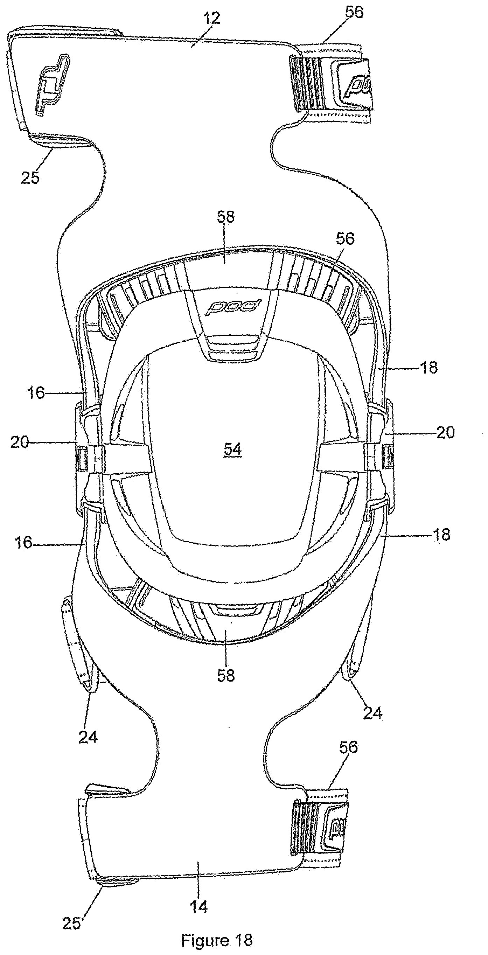

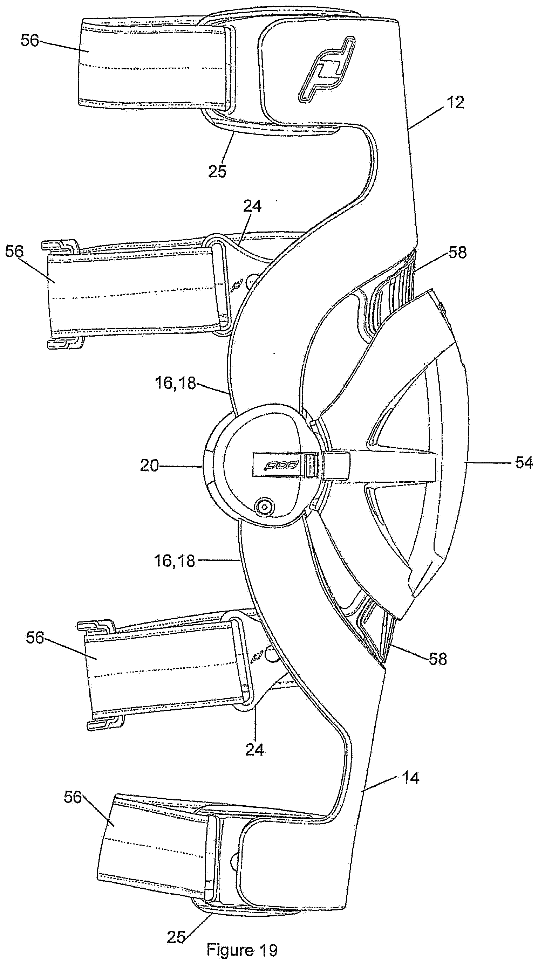

Referring to Figures 18 and 19, the knee brace 10 further includes upper and lower guard flaps 58 respectively removably connected to the upper and lower cuffs 12, 14 between the upper and lower pairs of struts 16, 18. The upper and lower guard flaps 58 are resiliently biased rearwardly to positively engage and position the upper and lower cuffs 12, 14 relative to a leg (not shown) above and below a knee joint. This ensures that the knee brace 10 remains correctly fitted to and placed against the leg during use. The guard flaps 58 are formed, for example, as integral mouldings in resilient plastics. Although not shown, the upper and lower guard flaps 58 are, for example, respectively removably connected to the upper and lower cuffs 12, 14 in similar fashion to the strap tabs 24 and the cuff adjustment tabs 25, namely by notch-pins 60 mounted, for example mouldingly embedded, in the upper and lower cuffs 12, 14 to respectively engage keyholes 64 in the upper and lower upper and lower guard flaps 58.

Although not shown, pads are respectively removably mounted, for example by hook and loop fasteners, internally of the joint capsules housings 20. Liners (not shown) are also respectively removably mounted, for example by hook and loop fasteners, rearwardly of the upper and lower cuffs 12, 14.

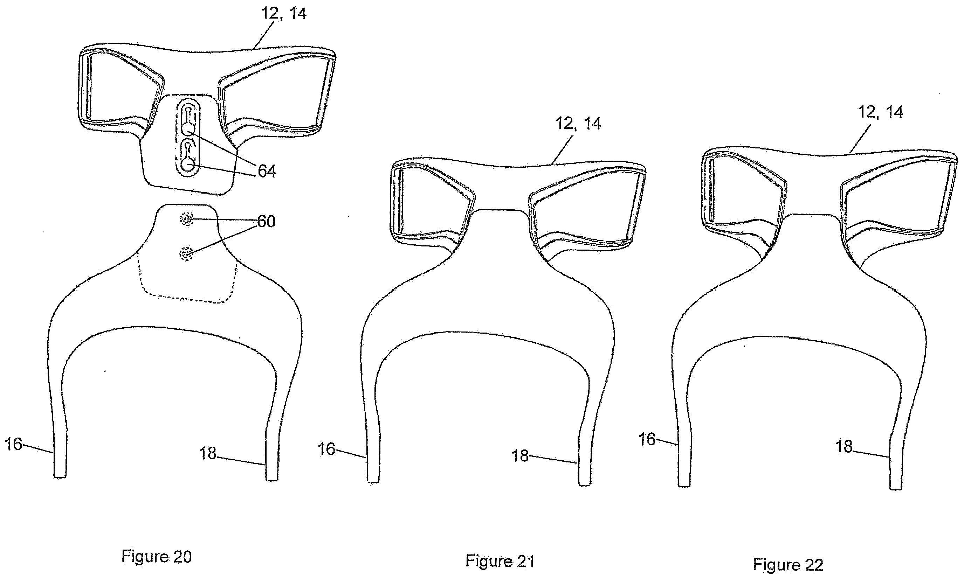

Referring to Figures 20 to 22, in another embodiment of the knee brace 10, the upper and lower cuffs 12, 14 are respectively removably connected to co-joined pairs of upper and lower struts 16, 18. The upper and lower cuffs 12, 14 are, for example, respectively removably connected to the co-joined pairs of upper and lower struts 16, 18 in similar fashion to the strap tabs 24, cuff adjustment tabs 25 and guard flaps 58, namely by notch-pins 60 mounted, for example mouldingly embedded, in the co-joined pairs of upper and lower struts 16, 18 to respectively engage keyholes 64 in the upper and lower cuffs 12, 14. The modular, interchangeable connection of the cuffs 12, 14 to the co-joined pairs of struts 16, 18 allows selective adjustment of:
(a) the overall length of the knee brace 10, as well as the respective lengths of the upper and lower subassemblies of the knee brace 10;
(b) the relative angles and curvatures of one or both of the upper and lower cuffs 12, 14 and/or one or both of the co-joined struts 16, 18; and
(c) the material compositions and/or mechanical properties of one or both of the upper and lower cuffs 12, 14 and/or one or both of the co-joined struts 16, 18.

For example, Figures 21 and 22 illustrate upper and lower cuffs 12, 14 respectively removably connected to different pairs of co-joined struts 16, 18 having mutually different lengths, namely the overall length of the co-joined struts 16, 18 in Figure 21 is shorter than that of Figure 22. This modular interchangeability allows the length of the knee brace 10 to be adjusted to fit different users and/or suit different uses. In addition, the cuffs 12, 14 and the co-joined pairs of struts can be formed from different materials having different mechanical properties depending on the intended use of the knee brace 10. For example, the co-joined pairs of struts 16, 18 are formed, for example as integral mouldings in rigid composite materials, and the upper and lower cuffs 12, 14 are formed, for example in rigid nylon or flexible urethane, depending on whether the intended use of the knee brace 10 requires rigid or flexible fitting of the upper and lower cuffs 12, 14 to the leg.

It will be appreciated that embodiments of the invention provide a knee brace having a simple modular construction that allows the fit and function of the knee brace to be easily adjusted and customised for different users and/or uses by simply interchanging different modular components.

The embodiments have been described by way of example only and modifications are possible within the scope of the invention disclosed.

## Claims

1. A system comprising:
a knee brace (10) including a strap (56); and
an apparatus for fastening the strap to the knee brace comprising:
a tab (24) including a keyhole slot (64) and an elongate slot (62); and
an embedded pin (60) protruding from the knee brace and terminating in an enlarged head;
wherein the keyhole slot comprises a first slot portion defining a first internal dimension adapted to receive the head of the pin, a narrow elongate second slot portion defining a second internal dimension less than the first internal dimension, and a third slot portion defining a third internal dimension less than the first internal dimension but greater than the second internal dimension whereby, upon traversing movement of the pin from the first slot portion to the third slot portion via the narrow elongate second slot portion, the tab is rotatably secured to the knee brace, and wherein when the system is in use the narrow elongate second slot portion inhibits traversing movement of the pin from the third slot portion back to the first slot portion; and
wherein the strap is threadable through the elongate slot and rotatably movable relative to the knee brace via the tab.

2. The system of claim 1, wherein the elongate slot is spaced apart from and perpendicular to the keyhole slot.

3. The system of claim 1 or claim 2, wherein the tab is made of plastic.

4. The system of any preceding claim, wherein the tab is generally triangular in shape.

5. The system of any preceding claim, wherein the narrow elongate second slot portion is substantially linear.

6. The system of any preceding claim, wherein the keyhole slot is positioned on a recessed surface of the tab.

7. The system of claim 6, wherein the recessed surface is generally triangular in shape.

8. The system of claim 6, wherein the elongate slot is positioned on an outer surface of the tab, wherein the outer surface is different than the recessed surface.

9. The system of claim 4, wherein the tab further comprises:
a first concave side positioned proximal to a second concave side; and a convex side positioned proximal to the first and second concave sides.

10. The system of claim 4, wherein the strap is fastened to the knee brace by the apparatus for fastening.
